# EUROPÄISCHE PATENTSCHRIFT

(11) **EP 2 510 101 B1**
(45) Veröffentlichungstag und Bekanntmachung des Hinweises auf die Patenterteilung: **27.09.2017**
(21) Anmeldenummer: 10785092.7
(22) Anmeldetag: 08.12.2010
(51) Int. Cl.: C12P 7/10, C12P 7/06, C07C 29/76

(54) **VERFAHREN ZUR GEWINNUNG VON ETHANOL WÄHREND EINER FERMENTATION**
METHOD FOR THE RECOVERY OF ETHANOL DURING FERMENTATION
PROCÉDÉ DE RECUPÉRATION D'ÉTHANOL PENDANT UNE FERMENTATION

(30) Priorität: 08.12.2009 EP 09178390
(43) Veröffentlichungstag der Anmeldung: 17.10.2012
(73) Patentinhaber: Süd-Chemie IP GmbH & Co. KG, 80333 München (DE)
(72) Erfinder: ZAVREL, Michael, 81379 München (DE); KRAUS, Michael, 82178 Puchheim (DE); HOFMANN, Sandra, 97285 Röttingen (DE); KETTLING, Ulrich, 81477 München (DE); KOLTERMANN, Andre, 82057 Icking (DE); OTT, Christian, 84539 Ampfing (DE); DRAGOVIC, Zdravko, 81375 München (DE)
(74) Vertreter: Graser, Konstanze
(86) Internationale Anmeldenummer: PCT/EP2010/069161
(87) Internationale Veröffentlichungsnummer: WO 2011/070061

(56) Entgegenhaltungen:
- US-A- 3 702 886
- US-A- 4 515 892
- US-A- 4 665 027
- HASHI M ET AL: "Ethanol recovery from fermentation broth via carbon dioxide stripping and adsorption", ENERGY AND FUELS, AMERICAN CHEMICAL SOCIETY, USA, Bd. 24, Nr. 9, 16. September 2010 (2010-09-16), Seiten 4628-4637, XP002634777, DOI: 10.1021/EF901130Q
- DATABASE ENERGY CITATIONS DATABASE 8. Februar 1983 (1983-02-08), Walsh P K et al: "Ethanol separation from water in a 2-stage adsorption process", XP002581550, Database accession no. 6838688 in der Anmeldung erwähnt & BIOTECHNOLOGY AND BIOENGINEERING SYMPOSIUM, NO. 13. 5TH SYMPOSIUM ON BIOTECHNOLOGY FOR FUELS AND CHEMICALS; GATLINBURG, TENN., USA, P. JOHN WILEY AND SONS, INC., NEW YORK, N.Y., USA., 10. Mai 1983 (1983-05-10), Seiten 629-647,
- VANE L M: "Separation technologies for the recovery and dehydration of alcohols from fermentation broths", BIOFUELS, BIOPRODUCTS AND BIOREFINING 2008 NOVEMBER/DECEMBER JOHN WILEY AND SONS LTD GBR LNKD- DOI:10.1002/BBB.108, Bd. 2, Nr. 6, November 2008 (2008-11), Seiten 553-588, XP002581551,
- DOMINGUEZ JOSE M ET AL: "Ethanol production from xylose with the yeast Pichia stipitis and simultaneous product recovery by gas stripping using a gas-lift loop fermentor with attached side-arm (GLSA)", BIOTECHNOLOGY AND BIOENGINEERING, Bd. 67, Nr. 3, 5. Februar 2000 (2000-02-05), Seiten 336-343, XP002581552, ISSN: 0006-3592 in der Anmeldung erwähnt
- DATABASE WPI Week 200830 Thomson Scientific, London, GB; AN 2008-E26493 XP002581553, & CN 101 024 846 A (UNIV FUDAN) 29. August 2007 (2007-08-29)
- DATABASE EPODOC [Online] EUROPEAN PATENT OFFICE, THE HAGUE, NL; 17. September 2008 (2008-09-17), XP002581554, Database accession no. CN-200810036646-A & CN 101 265 159 A (SHANGHAI LEAD BIOTECHNOLOGY CO [CN]) 17. September 2008 (2008-09-17)
- DATABASE WPI Week 200873 Thomson Scientific, London, GB; AN 2008-M35536 XP002581555, & CN 101 225 017 A (UNIV FUDAN) 23. Juli 2008 (2008-07-23)
- Yang, R. T.: "Gas separation by adsorption processes", 1997, Imperial College Press, London, XP002581721, Seiten 1-8, 23-26, Seite 1 - Seite 8; Tabelle 2.3 Seite 23 - Seite 26
- CARTON A ET AL: "Selection of adsorbents to be used in an ethanol fermentation process. Adsorption isotherms and kinetics", BIORESOURCE TECHNOLOGY, ELSEVIER, GB, Bd. 66, Nr. 1, Oktober 1998 (1998-10), Seiten 75-78, XP002634776, DOI: 10.1016/S0960-8524(98)00048-0
- CAPUTO ET AL: "Modeling of water and ethanol adsorption data on a commercial zeolite-rich tuff and prediction of the relevant binary isotherms", MICROPOROUS AND MESOPOROUS MATERIALS, ELSEVIER SCIENCE PUBLISHING, NEW YORK, US, Bd. 105, Nr. 3, 15. September 2007 (2007-09-15), Seiten 260-267, XP022250497, ISSN: 1387-1811, DOI: 10.1016/J.MICROMESO.2007.04.018
- OUMI Y ET AL: "Binary mixture adsorption of water and ethanol on silicalite", STUDIES IN SURFACE SCIENCE AND CATALYSIS, ELSEVIER BV, NL, vol. 142, 1 January 2002 (2002-01-01), pages 1595-1602, XP008136091, ISSN: 0167-2991, DOI: 10.1016/S0167-2991(02)80329-9 [retrieved on 2007-05-20]
- ANDRES T AGUAYO ET AL: "Study of operating variables in the transformation of aqueous ethanol into hydrocarbons on an HZSM-5 zeolite", JOURNAL OF CHEMICAL TECHNOLOGY AND BIOTECHNOLOGY, WILEY, vol. 77, no. 2, 1 February 2002 (2002-02-01), pages 211-216, XP001577756, ISSN: 0268-2575, DOI: 10.1002/JCTB.540 [retrieved on 2002-01-04]
- COSTA ENRIQUE ET AL: "ETHANOL TO GASOLINE PROCESS: EFFECT OF VARIABLES, MECHANISM, AND KINETICS", INDUSTRIAL AND ENGINEERING CHEMISTRY / PROCESS DESIGN AND DEVELOPMENT, AMERICAN CHEMICAL SOCIETY, WASHINGTON, DC, US, vol. 24, no. 2, 1 April 1985 (1985-04-01), pages 239-244, XP008105797, ISSN: 0019-7882, DOI: 10.1021/I200029A003

## Beschreibung

### Gebiet der Erfindung

Die Erfindung betrifft ein Verfahren zur Herstellung von Ethanol durch Fermentation.

### Hintergrund der Erfindung

Bei der Fermentation kohlenhydrathaltiger Rohsubstrate können Hefen oder Bakterien die Zuckermonomere bestehend aus fünf ("C5-Zucker", Pentosen) und / oder aus sechs Kohlenstoffatomen ("C6-Zucker", Hexosen) zu Ethanol umsetzen (Huber et al., Chem. Rev., 2006, Vol. 106, S. 4044-4098). Ethanol wird als "Bioethanol" bezeichnet, sofern es aus biogenen Rohstoffen hergestellt wird. Bioethanol eignet sich als Biokraftstoff, als Beimischung zu Ottokraftstoffen oder zur chemischen Weiterverarbeitung und wird bisher hauptsächlich aus Zucker sowie stärkehaltigem Getreide gewonnen, bislang jedoch nicht in nennenswerten Mengen aus lignocellulosischer Biomasse (LCB) (Huber et al., Chem. Rev., 2006, Vol. 106, S. 4044-4098; Kamm und Kamm, Chem. Ing. Tech., 2007, Vol. 79, S. 592-603).

Für die Herstellung von Ethanol durch Fermentation ist eine Trennung des Ethanols von der Fermentationslösung erforderlich. Zu diesen Techniken zählen Pervaporation, Extraktion, Adsorption, Umkehrosmose und Gas-Stripping (Windsperger et al., Verfahrenstechnik, 1989, Vol. 23, S. 16-21; Qureshi et al., Bioprocess Biosyst. Eng., 2005, Vol. 27, S. 215-222). Insbesondere kann das Ethanol in die Gasphase überführt werden. Gas-Stripping ist eine selektive Entfernung volatiler Substanzen aus der Fermentationslösung (Ezeji et al., J. Ind. Microbiol. Biotechnol., 2007, Vol. 34, 771-777).

Das Ethanol muss nach der Überführung in die Gasphase von dem Ethanol-Gas-Gemisch abgetrennt werden. Insbesondere muss das Ethanol nach dem Strippen von dem Ethanol-Trägergas-Gemisch abgetrennt werden. Hierfür stehen unterschiedliche Techniken zur Verfügung, wie z.B. die Kondensation oder selektive Adsorption des Ethanols an einen Adsorber. So beschreibt CA 1 195 258 ein Verfahren, bei dem nach erfolgter Fermentierung die Fermentationslösung einem Gas-Stripping unterworfen und das Ethanol-Trägergas-Gemisch dann an einem Molekularsieb unter Bedingungen adsorbiert wird, bei denen die Kapillarkondensation von Wasser vermieden wird. Dieses Verfahren erlaubt aber nicht, die Ethanolkonzentration während der Fermentation zu kontrollieren.

Eine solche Kontrolle der Ethanolkonzentration in der Fermentationslösung ist jedoch für eine industrielle Bioethanolherstellung wichtig. Ein Problem bei der Produktion von Bioethanol stellt die zunehmende inhibierende Wirkung und der toxische Einfluss des gebildeten Ethanols auf die Mikroorganismen während der Fermentation dar. Aufgrund der inhibierenden Wirkung und des toxischen Einflusses von während der Fermentation gebildeten Produkten wurden diverse Techniken entwickelt, um diese *in-situ* während der Fermentation abzutrennen.

So beschreiben Walsh et al. (Biotechnology and Bioengineering Symp., Nr. 13, 1983, S. 629 - 647) ein Verfahren, bei dem C6-Zucker zu Ethanol fermentiert und das Ethanol *in situaus* dem Fermenter durch Gas-Stripping abgetrennt und an Aktivkohle adsorbiert wird. Dieses Verfahren erlaubt, die Ethanolkonzentration während der Fermentation in dem Bereich um 6% (w/v) einzustellen. Aufgrund der geringen Selektivität von Aktivkohle für Ethanol eignet sich Aktivkohle jedoch nicht für ein wirtschaftliches Verfahren.

Für die Ethanolherstellung aus lignocellulosischer Biomasse, die die Fermentation von C5-Zuckern erfordert, ist eine solche Kontrolle bei 6% (w/v) jedoch nicht ausreichend. Beispielsweise konnten Dominguez et al. (Biotech. Bioeng., 2000, Vol. 67, S. 336-343) zeigen, dass die Umsetzung von C5-Zuckern zu Ethanol mit der Hefe *Pichia stipitis* bei nur 2% (w/v) Ethanol inhibiert wird. Dominguez et al. haben daher ein Verfahren entwickelt, bei dem die Ethanolkonzentration während der Fermentation von Xylose unter 2% (% (w/v) gehalten werden kann, wobei nach erfolgtem *in situ*-Stripping in einem besonders ausgestalteten Fermenter mit Seitenarm das Ethanol an einem eisgekühlten Kondensator kondensiert wird.

### Zusammenfassung der Erfindung

Vor diesem Hintergrund bestand die Aufgabe, ein wirtschaftliches Verfahren zur Herstellung von Ethanol durch Fermentierung bereitzustellen, das eine hohe Ethanolausbeute bei Verwendung von Gemischen aus C5 und C6-Zuckern, wie sie z.B. aus lignocellulosehaltiger Biomasse erhalten werden, erlaubt.

Es wurde überraschenderweise gefunden, dass durch Kombination von *in situ*-Stripping und einem Zeolithadsorber nicht nur die Ethanolkonzentration in der Fermentationslösung über die gesamte Fermentationsdauer bei unter 5% (w/v) gehalten werden kann, sondern zudem die Verwendung des Zeolithadsorbers eine besonders energiesparende Verfahrensführung erlaubt. Es wird somit erfindungsgemäß bereitgestellt ein Verfahren zur Ethanol-Herstellung umfassend:
a. die fermentative Umsetzung von C5 und/oder C6-Zuckern zu Ethanol in einer Fermentationslösung;
b. die *in situ*-Entfernung des Ethanols durch Überführen in die Gasphase mit Hilfe eines Trägergases, wobei die Ethanol-Konzentration in der Fermentationslösung unter 5% (w/v) gehalten wird und wobei das Gas-Stripping in einer mit dem Fermenter verbundenen Gas-Stripping-Säule durchgeführt wird, die kontinuierlich mit der Fermentationslösung gespeist wird und deren Auslass wieder zurück in den Fermenter geführt wird;
c. Durchleiten des durch das Gas-Stripping entstandenen Ethanol-Trägergas-Gemisches durch einen Zeolith-Adsorber, wobei Ethanol aus dem Gasgemisch an einen Adsorber adsorbiert wird und wobei mehrere Ad-/Desorptionssäulen parallel und / oder in Reihe geschaltet werden und die Adsorption in einer Säule zugleich mit der Desorption in einer anderen Säule durchgeführt wird; und
d. die Desorption des adsorbierten Ethanols von dem Adsorber
wobei das Trägergas nach dem Austritt aus dem Adsorber in die in der Gas-Strippingsäule enthaltenen Fermentationslösung rückgeführt wird.

### Abbildungen

Abbildungen 1a und 1b zeigen Beispiele des Verfahrens mit Gas-Stripping im Fermenter (1a, Vergleichsbeispiel) und in einer externen Gas-Stripping-Säule (1b, Ausführungsbeispiel).
Abbildung 2 zeigt den Verlauf der Ethanolkonzentration während der Fermentation gemäß Vergleichsbeispiel 1A.
Abbildung 3 zeigt den Verlauf der Glucose-, Xylose und Ethanolkonzentration während der Fermentation gemäß Vergleichsbeispiel 1B.
Abbildung 4 zeigt den Verlauf der Glucose-, Xylose und Ethanolkonzentration während der Fermentation gemäß Vergleichsbeispiel 1C.
Abbildung 5 zeigt den Verlauf der Glucose-, Xylose und Ethanolkonzentration während der Fermentation gemäß Vergleichsbeispiel 1D.
Abbildung 6 zeigt einen Vergleich zwischen dem erfindungsgemäß verwendeten Zeolith und Aktivkohle bezüglich der Ethanolselektivität.
Abbildung 7 zeigt eine erfindungsgemäße Ausführungsform mit Revolverkonfiguration.

### Detaillierte Beschreibung der Erfindung

### Fermentation von C5- und C6-Zuckern

Für die Fermentation wird eine Lösung mit C5- und C6-Zuckern bereitgestellt. Vorzugsweise enthält die Lösung anfänglich weniger als 200 g/L Zucker, davon weniger als 100 g/L C6-Zucker, besonders bevorzugt weniger als 80 g/L C6-Zucker, ganz besonders bevorzugt weniger als 70 g/L C6-Zucker, und weniger als 100 g/L C5-Zucker, besonders bevorzugt weniger als 35 g/L C5-Zucker, ganz besonders bevorzugt weniger als 30 g/L C5-Zucker. In einer besonders bevorzugten Ausführungsform enthält die Lösung weniger als 120 g/L Zucker, wobei 90 oder mehr % der Zucker C6-Zucker sind. In einer weiteren, besonders bevorzugten Ausführungsform enthält die Lösung weniger als 120 g/L Zucker, wobei 90 oder mehr % der Zucker C5-Zucker sind. In einer weiteren, besonders bevorzugten Ausführungsform enthält die Lösung weniger als 200 g/L Zucker, bevorzugt weniger als 120 g/L Zucker, wobei 20 bis 40% der Zucker C5-Zucker und entsprechend 60 bis 80% C6-Zucker sind. Diese Lösung wird regelmäßig aus kohlenhydrathaltigen Rohsubstraten gewonnen. Es kann notwendig sein, diese Rohsubstrate mittels geeigneter Vorbehandlungsverfahren aufzuschließen und / oder die Kohlenhydrate enzymatisch oder säurekatalysiert zu Zuckermonomeren zu hydrolysieren. Vor der Fermentation kann die Lösung optional aufkonzentriert werden.

Der Begriff "kohlenhydrathaltiges Rohsubstrat" umfasst kohlenhydrathaltige Reinstoffe, Gemische verschiedener Kohlenhydrate sowie komplexe Gemische von Substraten, die Kohlenhydrate enthalten. Kohlehydrathaltiges Material umfasst weiterhin Abfallprodukte aus der Forst- und Landwirtschaft und der Nahrungsmittel verarbeitenden Industrie sowie kommunale Abfälle. Unter die kohlenhydrathaltigen Materialien fällt insbesondere lignocellulosische Biomasse (LCB), welche Cellulose, Hemicellulose und Lignin enthält. LCB aus der Landwirtschaft umfasst unter anderem Getreidestroh und -spelzen (Weizen, Roggen, Gerste, Hafer), Maisstroh und -spindeln, Stallmist, Zuckerrohr-Presskuchen (Bagasse), Zuckerrüben-Pulpe (Zuckerrübenpressschnitzel) und krautige Materialien und Gräser, wie Gertengras, *Sericea Lespedeza,* Rutenhirse (Panicum virgatum, Switchgrass), Elephantengras (Miscanthus, Chinaschilf), und Sudangras (Sorghum sudananse, Sorghum drummondii). LCB in Form von Abfallprodukten aus der Forstwirtschaft umfasst unter anderem Baumrinde, Hackschnitzel und Holzverschnitt. LCB in Form von Rohsubstraten aus der Nahrungsmittelindustrie umfasst unter anderem Fruchtpulpe, Agavenrückstände, Kaffeerückstände und Ölmühlen-Abfälle, wie Rapssamen-Presskuchen und Mühlen-Abwässer. LCB in Form von Rohsubstraten aus der Zellstoff-und Papierindustrie umfasst unter anderem Papierbrei und Papiermühlen-Abwässer.

LCB in Form von Rohsubstraten aus kommunalen Abfällen umfasst, ist aber nicht beschränkt auf Papierabfälle, Gemüse- und Fruchtrückstände. Die Fermentationslösung wird bevorzugt aus LCB durch Hydrolyse erhalten. Der Lösung können weitere Zusatzstoffe wie pH-Stellmittel zugegeben werden.

Die bei der Hydrolyse freigesetzten C5- und/oder C6-Zucker, vorzugsweise C5-Zucker, gegebenenfalls zusammen mit C6-Zucker, werden durch Fermentation zu Ethanol umgesetzt. Nach der bevorzugten Ausführungsform der Erfindung kommen dabei Hefen oder Bakterien zum Einsatz. Besonders bevorzugt sind C5- und C6-Zucker metabolisierende Hefen und insbesondere solche, deren Fermentationsaktivität bei Ethanolkonzentrationen über 5% (% (w/v) inhibiert werden.

Nach dem erfindungsgemäßen Verfahren liegt die Temperatur des Fermenters zwischen 10 und 100°C, vorzugsweise zwischen 10 und 50°C, besonders vorzugsweise zwischen 20 und 50°C, ganz besonders vorzugsweise zwischen 20 und 40°C. Es werden bevorzugt mesophile Hefen verwendet, wie beispielsweise *Pichia stipitis, Pichia segobiensis, Candida shehatae, Candida tropicalis, Candida boidinii, Candida tenuis, Pachysolen tannophilus, Hansenula polymorpha, Candida famata, Candida parapsilosis, Candida rugosa, Candida sonorensis, Issatchenkia terricola, Kloeckera apis, Pichia barkeri, Pichia cactophila, Pichia deserticola, Pichia norvegensis, Pichia membranaefaciens, Pichia Mexicana* und *Torulaspora delbrueckii.* In einer alternativen Ausführung werden thermophile Mikroorganismen verwendet. Thermophile Hefen sind beispielsweise *Candida bovina, Candida picachoensis, Candida emberorum, Candida pintolopesii, Candida thermophila, Kluyveromyces marxianus, Kluyveromyces fragilis, Kazachstania telluris, Issatchenkia orientalis* und *Lachancea thermotolerans.* Thermophile Bakterien sind unter anderem *Clostridium thermocellum, Clostridium thermohydrosulphuricum, Clostridium thermosaccharolyticium, Thermoanaerobium brockii, Thermobacteroides acetoethylicus, Thermoanaerobacter ethanolicus, Clostridium thermoaceticum, Clostridium thermoautotrophicum, Acetogenium kivui, Desulfotomaculum nigrificans,* und *Desulfovibrio thermophilus, Thermoanaerobacter tengcongensis, Bacillus stearothermophilus und Thermoanaerobacter mathranii.* Besonders bevorzugt werden folgende mesophile Hefen verwendet: *Saccharomyces cerevisiae, Pichia stipitis, Pachysolen tannophilus, Candida shehatae*

Die Fermentation erfolgt vorzugsweise im Batch-Betrieb (absatzweise), im fed-batch-Betrieb oder im kontinuierlichen Betrieb. Besonders bevorzugt erfolgt die Fermentation im Batch-Betrieb.

### Das in-situ Stripping

Gemäß der vorliegenden Erfindung erfolgt *in-situ* eine Überführung der flüchtigen Bestandteile, insbesondere des Produkts Ethanol, in die Gasphase, insbesondere durch Strippen mit einem inerten Trägergas.

*In-situ*-Entfernung des Ethanols bezeichnet dabei die Entfernung des Ethanols, durch Gas-Stripping, parallel zu dessen fermentativer Herstellung. In der kontinuierlichen Betriebsweise wird gleichzeitig Zucker in Ethanol umgesetzt und mindestens ein Teil dieses Ethanols, beispielsweise durch Gas-Stripping, entfernt.

Für die Überführung mittels Gas-Stripping wird ein Trägergas verwendet. Als Trägergas kommen Gase wie Kohlendioxid, Helium, Wasserstoff, Stickstoff, oder Luft in Frage, sowie Mischungen dieser Gase. Besonders bevorzugt sind Kohlendioxid und Mischungen aus Kohlendioxid und Luft, wodurch bei Bedarf mikroaerobe Bedingungen eingestellt werden können. Ein Vorteil dieser Ausführungsform des erfindungsgemäßen Verfahrens besteht darin, dass das während der Fermentation gebildete Kohlendioxid direkt als Trägergas verwendet werden kann.

Entsprechend des erfindungsgemäßen Verfahrens erfolgt die Fermentation in einem Rührkessel-, oder in einem Schlaufenreaktor oder in einem Air-Lift-Reaktor. Zudem wird der Gasaustausch gemäß dem erfindungsgemäßen Verfahren über eine mit dem Fermenter verbundene, externe Gas-Stripping-Säule durchgeführt, die kontinuierlich mit der Fermentationslösung gespeist wird und deren Auslass wieder zurück in den Fermenter geführt wird. Besonders bevorzugt wird eine solche externe Gas-Stripping-Säule im Gegenstrom und/oder in Kombination mit Füllkörpern für einen erhöhten Stoffaustausch, wie z.B. Raschigringen betrieben.

Die spezifische Begasungsrate liegt vorzugsweise zwischen 0,1 und 10 vvm, besonders bevorzugt zwischen 0,5 und 5 vvm.

Das Stripping wird vorzugsweise bei einem Druck zwischen 0,1 und 2 bar, besonders bevorzugt zwischen 0,5 und 1,1 bar, durchgeführt. Besonders bevorzugt ist ein Stripping bei Unterdruck.

Um ein effizientes Gas-Stripping im Fermenter zu erzielen, werden die Gasblasen vorzugsweise dispergiert. Dies kann mit einem Rührer erfolgen, der so angeordnet ist, dass feine Blasen des Trägergases entstehen.

In einer bevorzugten Ausführungsform erfolgt die *in-situ-Entfernung* von Ethanol aus der Fermentationslösung bei der Fermentationstemperatur. Somit ist keine zusätzliche thermische Energie für die Erwärmung der Fermentationslösung notwendig.

Ein weiterer Vorteil des erfindungsgemäßen Verfahrens besteht darin, dass die durch den Übergang der flüchtigen Substanzen von der Flüssigkeit in die Gasphase abgeführte Verdampfungsenthalpie zur Kühlung des Fermenters beiträgt und somit die erforderliche Kühlleistung zur Konstanthaltung der Temperatur im Fermenter verringert wird.

### Die Adsorption

Gemäß dem erfindungsgemäßen Verfahren wird der den Fermenter verlassende Gasstrom durch eine oder mehrere Säulen geleitet, welche mit einer oder mehreren Arten von Adsorbentien gefüllt sind. Zumindest eine der Säulen enthält Zeolith als Adsorber. Als weitere Adsorbentien eignen sich Silica, Bentonite, Silicalite, Tone, Hydrotalcite, Aluminiumsilikate, Oxidpulver, Glimmer, Gläser, Aluminate, Clinoptolite, Gismondine, Quartze, Aktivkohlen, Knochenkohle, Montmorillonite, Polystyrole, Polyurethane, Polyacrylamide, Polymethacrylate oder Polyvinylpyridine. In einer besonders bevorzugten Ausführungsform werden ausschließlich Zeolithe als Adsorbentien verwendet.

Bevorzugt sind Zeolithe, besonders bevorzugt Zeolithe des beta- oder MFI-Typs. Vorzugsweise weist der Zeolith ein SiO₂/Al₂O₃-Verhältnis von 200 bis 1000 auf, und besonders bevorzugt ein SiO₂/Al₂O₃-Verhältnis von 400 bis 800. Besonders bevorzugt sind die synthetischen Zeolithe gemäß US 7,244,409.

Das Massenverhältnis von Adsorbens zu adsorbierten Ethanol liegt vorzugsweise zwischen 1 und 1000, besonders bevorzugt zwischen 5 und 20.

Bei der Adsorption von Ethanol an das Adsorbens (die Adsorbentien) wird die Adsorptionsenthalpie freigesetzt, welche zu einer Erhitzung der Packung führt. Aufgrund der geringen Wärmeleitfähigkeit der beschriebenen möglichen Adsorbensmaterialien und des Hohlraumvolumens innerhalb der Schüttung kann diese Wärme insbesondere bei großen Säulendurchmessern nicht effektiv über die Säulenwand abgeführt werden. In einer bevorzugten Ausführungsform der vorliegenden Erfindung kommen deshalb zusätzlich Heizwendeln innerhalb der Säule zum Einsatz, welche den Austrag der freiwerdenden Adsorptionsenthalpie ermöglichen.

Ein Vorteil dieser Ausführungsform besteht darin, dass somit Energie für die nachfolgenden energieerfordernden Prozessschritte gewonnen werden kann.

Als Heizwendeln eignen sich Rohre, die mit einem Fluid durchströmt werden und die somit sowohl den Ein- als auch Austrag von thermischer Energie ermöglichen. Alternativ sind elektrisch beheizbare Heizwendeln einsetzbar.

Entsprechend dem erfindungsgemäßen Verfahren kann über die Heizwendeln innerhalb der Säule die Temperatur beeinflusst und konstant gehalten werden, wodurch die Selektivität des Adsorbens beeinflusst werden kann. In einer bevorzugten Ausführungsform des Verfahrens wird die Selektivität nicht nur durch die Temperatur, sondern auch durch den Druck innerhalb der Säule gesteuert.

Die Temperatur bei der Adsorption des Ethanols liegt vorzugsweise zwischen 10-100°C, besonders bevorzugt zwischen 20 und 50°C. Der Druck liegt vorzugsweise zwischen 0,5-10 bar, besonders bevorzugt zwischen 1 und 2 bar liegt.

Besonders bevorzugt ist es, die Adsorption bei einer Temperatur durchzuführen, die nicht über der Temperatur des Ethanol-Trägergas-Gemisches bei Austritt aus der Fermentationslösung liegt. In einer besonders bevorzugten Ausführungsform werden weder das Ethanol-Gas-Gemisch noch der Adsorber vor der Adsorption erwärmt. Zudem ist es besonders bevorzugt, dass die Adsorption bei Überdruck erfolgt.

In einer bevorzugten Ausführungsform ist bei diesem Verfahren mindestens ein C5-Zucker in der Fermentationslösung vorhanden. Dies schließt auch Fermentationslösungen ein, die Mischungen von mindestens einem C5-Zucker und mindestens einem C6-Zucker umfassen. Es ist besonders bevorzugt, dass der in der Fermentationslösung vorhandene mindestens eine C5-Zucker zu Ethanol umgesetzt wird.

Die Menge des verwendeten Adsorbermaterials wird vorzugsweise an die Menge des durch die Fermentation entstehenden Ethanols angepasst. Vorzugsweise beträgt die Menge adsorbierten Ethanols am Ende der Fermentation mindestens 20%, vorzugsweise mindestens 50% und besonders bevorzugt mindestens 90% der maximalen Ethanolaufnahmemenge des Adsorbers. Sowohl die durch die Fermentation entstehende Ethanolmenge wie auch die maximale Ethanolaufnahmemenge des Adsorbers können vor der Fermentation bestimmt werden. Für die Bestimmung beider Größen erfolgt das Gas-Stripping und die Adsorption genauso wie in Beispiel 2 beschrieben, d.h. eine Lösung mit bekannter Ethanol-Konzentration wird vorgelegt und dann kontinuierlich gestrippt. Während dieser Zeit wird die Ethanol-Konzentration in der Vorlage stündlich gemessen. Wenn sich diese nicht mehr ändert (spätestens nach 24 Stunden) ist die Kapazität des Adsorbermaterials erschöpft. Dann wird der Versuch beendet und das Volumen der Vorlage und die Konzentration des darin erhaltenen Ethanols bestimmt, so dass die Massen von Ethanol und Wasser berechnet werden können. Die Differenzen zwischen den anfangs vorgegebenen Massen und denen nach Versuchsende ergeben die adsorbierten Massen von Ethanol und Wasser (Massenbilanz). Mit Hilfe dieser Werte lassen sich die adsorbierte Ethanol-Konzentration und die Kapazität des Adsorbermaterials bestimmen. Die maximal in der Fermentation gebildete Ethanolmenge kann mit Hilfe der theoretischen Ausbeutekoeffizienten abgeschätzt werden. Die theoretischen Ausbeutekoeffizienten liegen bei 0,51 g Ethanol pro 1 g Glucose beziehungsweise 0,46 g Ethanol pro 1 g Xylose (Lee et al., J. Microbiol. Biotechn., 2001, Vol. 11 (3), S. 384-388). Die in der Praxis erreichten Ethanol-Ausbeuten liegen bei 70-100%, typischerweise bei 90-95% der theoretischen Ausbeuten. Mit Hilfe der zu erwartenden Ethanolmenge und einem Zuschlag von typischerweise 10-20% erfolgt die Berechnung der benötigten Adsorbermenge.

Das Adsorbermaterial ist in mehreren Säulen enthalten. Vorzugsweise werden mehrere, besonders bevorzugt 2 bis 6 Säulen verwendet. Diese Säulen sind in Reihe oder parallel geschalten.

Vorteile der Parallelschaltung sind zum einen, dass somit ein quasi-kontinuierlicher Betrieb ermöglicht wird, indem zwei oder mehr Säulen zwischen Ad- und Desorption alternieren, und zum anderen, dass die freiwerdende thermische Energie bei der Adsorption zur Desorption in einer anderen Säule übertragen werden kann, also Adsorption und Desorption zeitgleich an unterschiedlichen Säulen durchgeführt werden können. Die Säulen werden vorzugsweise in einer Revolveranordnung bereitgestellt. In einer besonders bevorzugten Ausführungsform werden 2 bis 6 Säulen so geschaltet, dass die Säule bzw. Säulen, in denen die Adsorption abläuft, parallel zu der bzw. den Säulen geschaltet wird, in denen die Desorption abläuft. Läuft in mehr als einer Säule die Adsorption ab, so können diese Säulen in Reihe geschaltet werden. So kann bei Verwendung von 6 Säulen z.B. in der "Revolver"-Konfiguration" in den Säulen 1 bis 3 die Adsorption ablaufen, Säule 4 für die Desorption erwärmt werden, in Säule 5 die Desorption ablaufen, und Säule 6 lässt man auskühlen. Die Adsorbersäule wird gewechselt, wenn die Menge adsorbierten Ethanols mindestens 90%, besonders bevorzugt mindestens 95% der maximalen Ethanolaufnahmemenge des Adsorbers in dieser Säule beträgt.

Das erfindungsgemäße Verfahren mit mehreren Adsorptionssäulen eröffnet zudem die Möglichkeit, zwei oder mehr Säulen in Reihe zu schalten, die jeweils mit unterschiedlichen Adsorbentien gefüllt sind, die unterschiedliche Selektivitäten und / oder Kapazitäten aufweisen. Hierbei durchströmt vorzugsweise das Trägergas bei der Adsorption die Adsorber in Reihenfolge steigender Ethanol-Bindeselektivitäten (in Bezug auf Wasser).

Der an Ethanol abgereicherte Gasstrom kann nach Austritt aus der Adsorptionssäule zurück in die in der Gas-Stripping -Säule enthaltenen Fermentationslösung geführt werden und steht dann erneut für das Gas-Stripping zur Verfügung.

Die Adsorption kann im Wirbelschichtbetrieb durchgeführt werden.

Mit der erfindungsgemäßen Kombination aus *in-situ*-Gas-Stripping und Adsorption an Zeolith kann die Ethanolkonzentration in der Fermentationslösung auf unter 5% (w/v), vorzugsweise unter 2% (w/v) über die gesamte Fermentationsdauer gehalten werden. Die Fermentation wird vorzugsweise durchgeführt, solange Ethanol entsteht.

Bevorzugte Fermentationsdauern betragen 20 bis 120 Stunden, besonders bevorzugt 30 bis 80 Stunden.

### Die Desorption

Das erfindungsgemäße Verfahren ermöglicht die selektive Desorption des Ethanols vom Adsorbens durch Erhöhung der Temperatur und / oder Verringerung des Drucks innerhalb der Säule. In einer bevorzugten Ausführungsform des Verfahrens wird die thermische Energie über die Säulenwand und ggf. zusätzlich über die Heizwendeln im Inneren der Säule direkt auf die Adsorbenspackung eingebracht. Bevorzugt sind Temperaturen zwischen 25 und 300°C und Absolutdrücke zwischen 0 und 10 bar. Besonders bevorzugt sind Temperaturen zwischen 80 und 180°C, sowie Absolutdrücke bei Unterdruck, vorzugsweise zwischen 0,1 und 1 bar.

Entsprechend des erfindungsgemäßen Verfahrens wird für den Austrag des desorbierten Ethanols aus der Säule ein Trägergas verwendet. Bevorzugt wird hierbei das gleiche inerte Trägergas verwendet, welches auch für das Gas-Stripping eingesetzt wird. In einer Ausführungsform des erfindungsgemäßen Verfahrens werden die Temperatur und der Absolutdruck des Trägergases entsprechend den oben beschriebenen Temperaturen und Absolutdrücken innerhalb der Säule eingestellt. Für diesen Zweck eignen sich vorgeschaltene Wärmetauscher und / oder Drosseln beziehungsweise Kompressoren.

Die Desorption kann im Wirbelschichtbetrieb durchgeführt werden.

### Weitere Aufreinigung

Eine bevorzugte Ausführungsform des erfindungsgemäßen Verfahrens sieht die Kondensation des desorbierten Ethanol-Gases vor. Nach einer bevorzugten Ausführungsform des Verfahrens wird dazu der Gasstrom komprimiert und / oder gekühlt durch Verwendung von einem oder mehreren Kompressoren und / oder einem oder mehreren Wärmetauschern und / oder einer oder mehreren Kühlfallen. Besonders bevorzugt sind Gegenstromwärmetauscher. In einer weiteren bevorzugten Ausführungsform des erfindungsgemäßen Verfahrens werden durch die Reihenschaltung von zwei oder mehreren Wärmetauschern und / oder Kühlfallen mit unterschiedlichen Kühltemperaturen Kondensate mit unterschiedlichen Ethanol-Konzentrationen erhalten. Zudem eröffnet dies die Möglichkeit der selektiven Kondensation noch vorhandener weiterer Begleitstoffe, wie Wasser oder anderen flüchtiger Substanzen.

Bei der Kondensation wird die Kondensationsenthalpie freigesetzt. Gemäß einer bevorzugten Ausführungsform des erfindungsgemäßen Verfahrens wird diese thermische Energie mittels Wärmetauschern auf vorausgehende und / oder mögliche nachfolgende, energieerfordernde Verfahrensschritte übertragen. Gemäß einer besonders bevorzugten Ausführungsform des erfindungsgemäßen Verfahrens handelt es sich bei diesen energieerfordernden Verfahrensschritten um die vorangegangene Desorption des Ethanols und / oder eine mögliche nachfolgende Rektifikation.

Entsprechend einer weiteren Ausführungsform des erfindungsgemäßen Verfahrens wird das erhaltene kondensierte Ethanol weiter aufgereinigt und aufkonzentriert. Ein typischer Begleitstoff des Ethanols im Kondensat ist Wasser. Die Entfernung von Wasser und / oder weiterer Begleitstoffe kann durch Rektifikation erfolgen.

In einer bevorzugten Ausführungsform des Verfahrens wird bei der Kondensation des Ethanols die Temperatur knapp unterhalb des Siedepunkts des entstehenden Kondensats gehalten, so dass die zu rektifizierende Ethanol-Lösung nahe am Siedepunkt an die Rektifikation übergeben wird, wodurch der Energieaufwand für die Rektifikation reduziert wird. Mittels Brüdenkompression kann der Energiebedarf der Rektifikation noch weiter reduziert werden.

Das im Sumpf der Rektifikationskolonne erhaltene Wasser kann in den Fermenter zurückgeführt werden. Am Kopf der Kolonne wird das Azeotrop zwischen Ethanol und Wasser erhalten. Um wasserfreies Ethanol zu erhalten, ist es möglich nachfolgend geeignete Trennverfahren einzusetzen, wie zum Beispiel die Entfernung von Wasser mittels eines Molekularsiebs oder durch Einsatz selektiver Membranverfahren. Ebenso ist es möglich die Lage des Azeotrops über die Veränderung des Drucks bei der Rektifikation zu verschieben.

Entsprechend einer alternativen Ausführungsform des erfindungsgemäßen Verfahrens wird vor der Kondensation des desorbierten Ethanol-Gases dieses über eine weitere Säule zur Entfernung von Begleitstoffen aus der Gasphase geleitet. Bevorzugt ist hierbei die Entfernung von Wasser mittels eines Molekularsiebs. Außerdem ist bei dieser alternativen Ausführungsform die Anwendung von Dampfpermeation möglich.

Eine weitere alternative Ausführungsform des erfindungsgemäßen Verfahrens sieht vor, das nach der Desorption erhaltene Kondensat einer Pervaporation zuzuführen, so dass absolutes Ethanol erreicht werden kann.

Gemäß dem erfindungsgemäßen Verfahren wird der Trägergasstrom, der durch die Kondensation des Ethanols und anderer möglicher Begleitstoffe rückgewonnen wird, im Kreislauf geführt, so dass für das Gas-Stripping im Fermenter keine oder eine nur geringe externe Zufuhr von Trägergas notwendig ist.

### Besonders bevorzugte Ausführungsformen

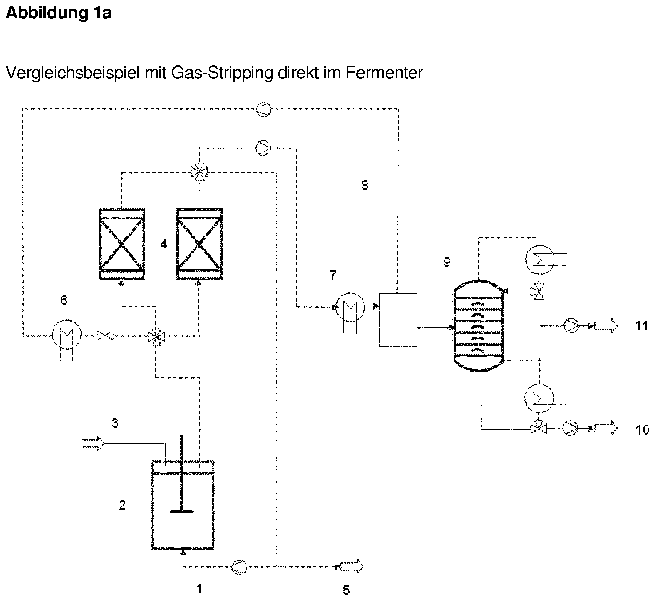

Abbildung 1a zeigt eine allgemeine Ausführungsform eines Strippingverfahrens Verfahrens. Ein inerter Trägergasstrom (1) wird zum Gas-Stripping in den Fermenter (2) eingeblasen. Innerhalb des Fermenters wird LCB zu Ethanol fermentiert, wobei Hilfsstoffe (3) wie pH-Stellmittel zugegeben werden.

Das den Fermenter verlassende Gas, welches Ethanol und andere flüchtige Bestandteile enthält, wird durch eine Adsorptionssäule (4) geleitet, die das Ethanol selektiv adsorbiert. Um einen quasi-kontinuierlichen Betrieb zu gewährleisten, werden zwei oder mehrere Säulen parallel und/oder in Reihe geschaltet. Ein thermischer Austausch zwischen den Säulen wird durch interne Heizwendeln erreicht.

Ein Teil des Trägergasstroms wird aufgrund des fermentativ gebildeten Kohlenstoffdioxids abgeführt (5).

Zur Desorption des adsorbierten Ethanols wird die Temperatur und / oder der Druck innerhalb der Säulen (4) verändert. Der für den Austrag des desorbierten Ethanols notwendige Trägergasstrom wird über einen Wärmetauscher (6) und / oder Drosseln entsprechend eingestellt.

Das bei der Desorption die Säule verlassende Gas wird anschließend mittels Kompression und / oder Kühlung kondensiert (7). Der somit regenerierte Trägergasstrom (8) wird rückgeführt.

Das Kondensat wird weiter aufgereinigt und aufkonzentriert, indem dieses einer Rektifikationskolonne (9) zugeführt wird. Am Sumpf der Kolonne wird Wasser erhalten (10), am Kopf der Kolonne das Azeotrop zwischen Ethanol und Wasser (11).

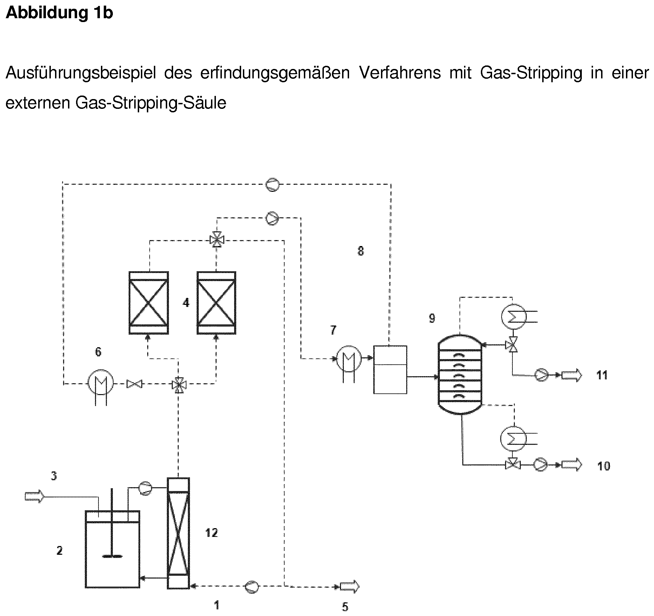

Abbildung 1b zeigt eine mögliche Ausführungsform des erfindungsgemäßen Verfahrens, wobei in diesem Fall das Gas-Stripping in einer mit dem Fermenter verbundenen externen Gas-Stripping-Säule (12) erfolgt. Dabei wird Fermentationslösung der externen Gas-Stripping-Säule zugeführt und die gestrippte Lösung dann wieder zurück in die in der Gast-Stripping-Säule enthaltene Fermentationslösung geführt. Alle weiteren Verfahrensschritte sind analog zu Abbildung 1a.

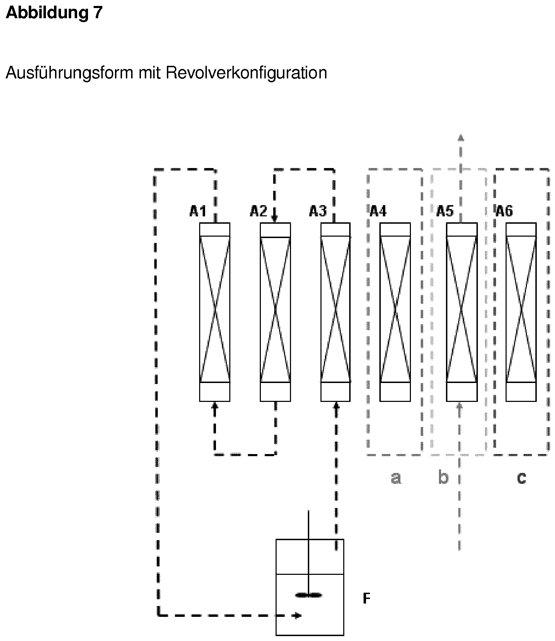

Abbildung 7 zeigt die "Revolverkonfiguration, bei der drei Säulen (A1-A3) für die Adsorption aus dem vom Fermenter (F) stammenden Stripping-Gas in Reihe geschaltet sind. Die Säulen A4 bis A6 sind parallel geschaltet. Die Säule A4 wird erwärmt (a), die Säule A5 desorbiert (b) und die Säule A6 abgekühlt (c). Nach Ende der Taktzeit gelangt Säule A3 in die Erwärmungsphase (a), A4 wird desorbiert (b) und A5 abgekühlt (c). Für die Adsorption sind dann die Säulen A6, A1 und A2 in Reihe geschaltet. Nach 6 Taktzeiten wird wieder dieselbe Säule desorbiert wie zu Beginn, so dass ein Zyklus vollendet ist und ein neuer beginnt.

### Vergleichsbeispiele

### Vergleichsbeispiel 1- In-situ-Abtrennung von Ethanol während der Fermentation

### A) Fermentation mit Pachysolen tannophilus in synthetischem Medium

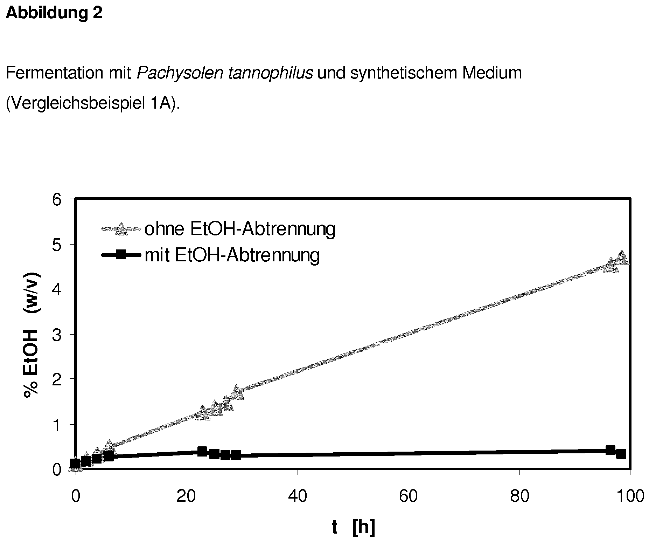

*Pachysolen tannophilus* (DSMZ, Braunschweig) wurde mit und ohne *in-situ*-Abtrennung von Ethanol unter ansonsten identischen Bedingungen für 100 Stunden bei 30°C fermentiert. Das Fermentationsmedium bestand aus 5 g/L Bacto TM Yeast Extract (Becton, Dickinson Co., Frankreich), 6,7 g/L 1 x Difco Yeast Nitrogen Base w/o Amino Acids (Becton, Dickinson Co., Frankreich), auf 350 mL H₂O dest. Zwei je 350 mL Kulturen wurden in 1 L Schott-Glasflaschen mit gasdichten GL 45 Flaschen-Mehrfachverteiler (Bola, Grünsfeld), die drei GL14 Zugänge für Schlauchverschraubungen hatten, angesetzt. Als Kohlenstoffquelle wurde jeweils 35 g Glucose im Fed-Batch-Betrieb verwendet. Ein Zugang wurde zur Probenabnahme verwendet. Durch einen weiteren Zugang wurde das Trägergas (Stickstoff) über einen PA 12-8x6x1 Schlauch (Riegler, Bad Urach) und eine Glasfritte in das Fermentationsmedium eingeleitet. Über den dritten Zugang wurde das Trägergas in einem Schlauch vom Flaschenkopf in eine 100 mL Glasfrittensäule, die für die *in-situ-*Abtrennung mit einem Zeolith (Hergestellt nach US Patent No. 7,244,409 B2) gefüllt ist, geleitet. Für das Referenzexperiment war die Glasfrittensäule nicht gefüllt. Der Gaskreislauf wurde durch eine Membranpumpe (KNF, Freiburg), die zwischen die Glasfrittensäule und die Schott-Glasflaschen geschaltet wurde, mit 1,5 L/min betrieben. Je 2,5% (w/v) Glucose (Sigma-Aldrich, München) wurden bei 0, 24, 48 und 72 Stunden zugefüttert. Die Menge an Ethanol im Fermentationsmedium wurde mittels Gas-Chromatographie (Trace GC, Thermo Fisher) bestimmt. Das Ergebnis des Experiments ist in Abbildung 2 dargestellt. Die GC-Bestimmungen der Ethanol-Konzentrationen in den Medien zeigen, dass durch die *in-situ*-Abtrennung die Ethanol-Konzentration des Fermentationsmediums unter 1% (w/v) Ethanol gehalten werden kann. Inhibierungen durch die Ethanol-Konzentration werden somit vermieden.

Unter ansonsten identischen Bedingungen wie oben beschrieben, wurde weiterhin eine Mischung von C5- und C6-Zuckern mit und ohne *in-situ*-Abtrennung von Ethanol fermentiert. Dabei wurden 24,5 g Glucose und 10,5 g Xylose als Kohlenstoffquelle im Batch-Betrieb verwendet.

### B) Fermentation mit Pichia stipits auf lignocellulosischem Substrat

*Pichia stipitis* (DSMZ, Braunschweig, Deutschland) wurde mit und ohne *in-situ-*Abtrennung von Ethanol unter ansonsten identischen Bedingungen für 95 Stunden bei 30°C unter mikroaeroben Bedingungen fermentiert. Das Fermentationsmedium war vorbehandeltes und hydrolysiertes lignocellulosisches Substrat. Zwei je 800 mL Kulturen wurden in 1,4 L Kleinfermenter angesetzt. Durch Einsatz des lignocellulosischen Substrats waren als Kohlenstoffquelle jeweils 56 g/l Glucose und 31 g/L Xylose enthalten. Vergärt wurde im Batch-Betrieb. Eine der beiden Kultivierungen wurde ohne in-situ-Stripping, die andere mit in-situ-Stripping durchgeführt. Im letzteren Fall wurde der Gasstrom über ein Rotameter (Vöglin, Aesch, Schweiz) auf 1 vvm eingestellt. Mit Hilfe einer Membranpumpe (KNF, Freiburg, Deutschland) und gasdichten Schläuchen (VWR, Darmstadt, Deutschland) wurde der Gasstrom durch eine Glassäule geleitet und dann rückgeführt. Die Glassäule war mit 535 g Zeolith-Granulat (ZSM-5; SiO₂/Al₂O₃=200; Süd-Chemie AG, Deutschland) gepackt. Während der Fermentation wurden Proben genommen und der Ethanol-Gehalt gaschromatografisch (Trace GC, ThermoFisher, Deutschland) und die Zucker über HPLC (Dionex, USA) quantifiziert. Zudem wurde die Gewichtszunahme des Zeolithen und der Wasseranteil der adsorbierten Mischung über Karl-Fischer-Titration (Schott Instruments, Deutschland) bestimmt. Zudem wurde angenommen, dass lediglich Wasser und Ethanol unter den gegebenen Bedingungen adsorbiert werden, was in Vorversuchen bestätigt werden konnte. Dadurch kann aus dem Wassergehalt auf den Ethanolanteil rückgeschlossen werden.

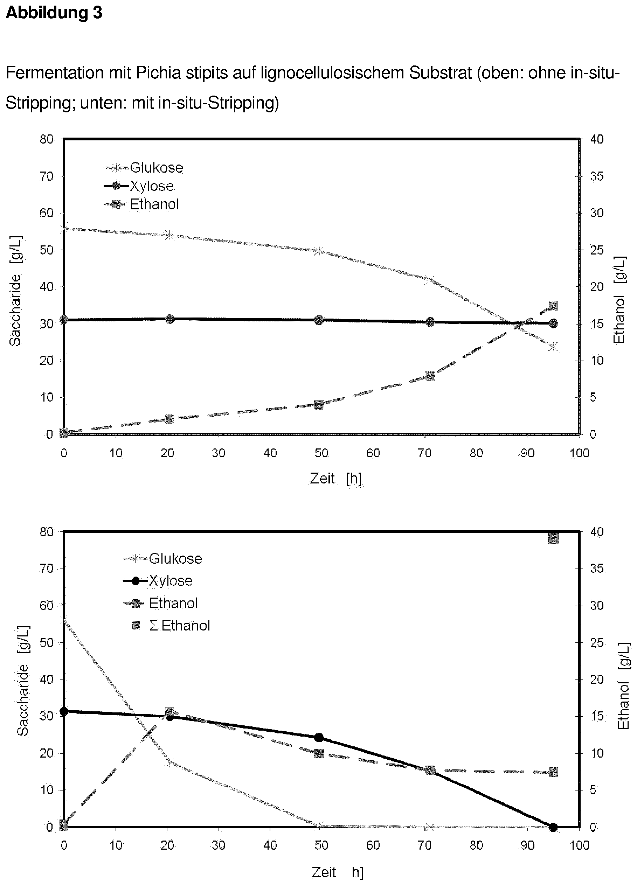

Die Ergebnisse des Experiments sind in Abbildung 3 (oben: ohne in-situ-Stripping; unten: mit in-situ-Stripping) dargestellt. Die Analysenwerte der Fermentationsbrühen zeigen, dass durch die *in-situ*-Abtrennung die Ethanolkonzentration des Fermentationsmediums unter 2% (w/v) Ethanol gehalten werden kann. Inhibierungen durch die Ethanol-Konzentration werden somit vermieden und die Vergärung des Xyloseanteils wird dadurch ermöglicht.

### C) Sequentielle Fermentation mit Saccharomyces cerevisiae und Pachysolen tannophilus auf lignocellulosischem Substrat

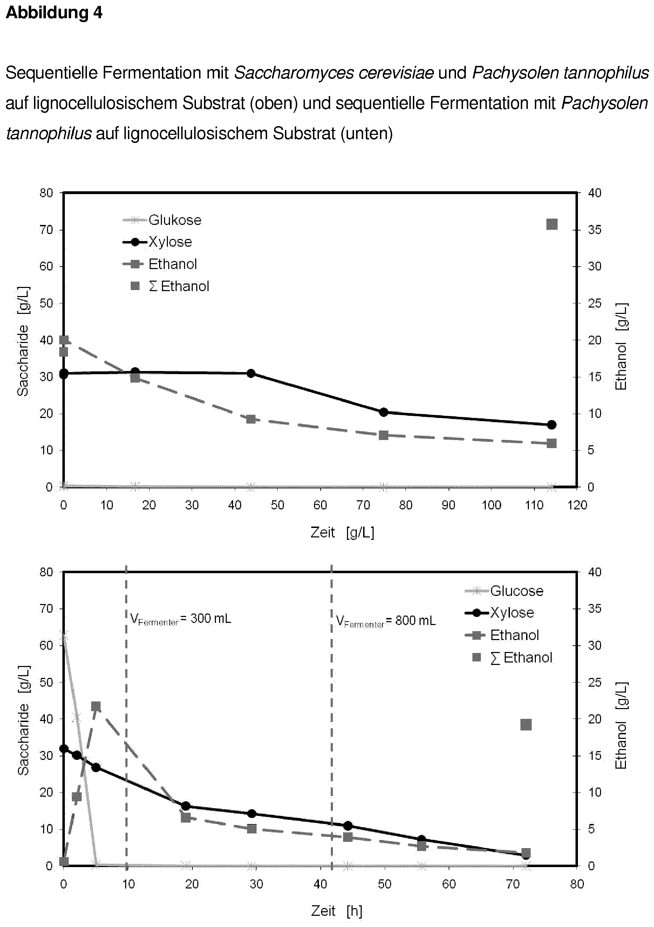

Wenn nicht anders angegeben, waren alle Bedingungen in diesem Experiment identisch mit Beispiel 1B. Es wurde eine sequentielle Vergärung durchgeführt, wobei zunächst die Vergärung der Glucose anaerob im Batch-Betrieb mit *Saccharomyces cerevisiae* (DSMZ, Braunschweig, Deutschland) ohne in-situ-Gas-Stripping durchgeführt wurde. In der eingesetzten Lösung waren 63 g/L Glucose und 32 g/L Xylose enthalten. Vergärt wurde im Batch-Betrieb. Das daraus gewonnene xylose- und ethanolhaltige Substrat wurde mit *in-situ*-Abtrennung von Ethanol unter mikroaeroben Bedingungen im Batch mit *Pachysolen tannophilus* (DSMZ, Braunschweig, Deutschland) für 114 Stunden bei 30°C fermentiert. Das Ergebnis dieser zweiten Fermentationsphase ist in Abbildung 4 (oben) dargestellt. Es wird deutlich, dass die C5-Vergärung erst einsetzt, wenn die Ethanol-Konzentration durch das Gas-Stripping auf Werte unterhalb von ca. 15 g/L reduziert wird. Erst durch das Gas-Stripping wird also die Vergärung der C5-Zucker ermöglicht.

In einem zweiten Experiment zur sequentiellen Vergärung erfolgte die Vergärung der Glucose im Batch-Betrieb mit *Pachysolen tannophilis* (DSMZ, Braunschweig, Deutschland). Das daraus gewonnene xylose- und ethanolhaltige Substrat wurde mit *in-situ*-Abtrennung von Ethanol unter mikroaeroben Bedingungen als Feed-Lösung im Fed-batch-Betrieb mit dem gleichen Organismus für 72 Stunden bei 40°C fermentiert. Das Startvolumen für die Fed-Batch-Kultivierung betrug 300 mL, wobei diese 300 mL nicht vorvergärt wurden. Die Ergebnisse der fed-batch-Phase sind in Abbildung 4 (unten, zwischen den beiden vertikalen Markierungslinien) dargestellt. Es ist erkennbar, dass durch die Kombination von fed-batch-Betriebsweise mit Gas-Stripping die Ethanol-Konzentration während der C5-Vergärung auf einem besonders niedrigen Niveau gehalten werden kann.

### D) Fed-batch-Fermentation mit Pachysolen tannophilus auf lignocellulosischem Substrat

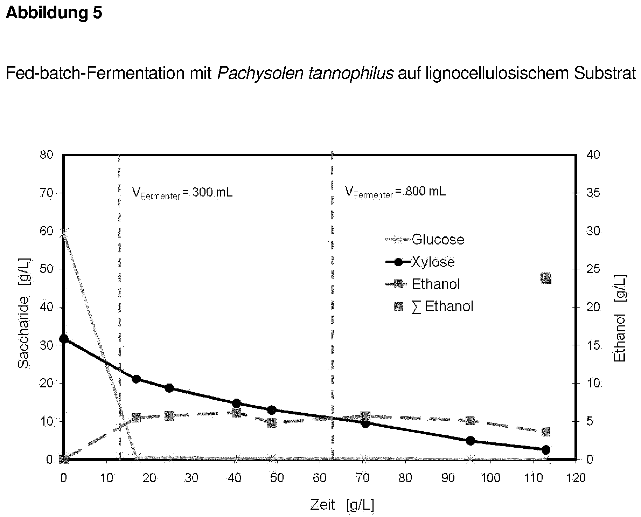

Wenn nicht anders angegeben, waren alle Bedingungen in diesem Experiment identisch mit Beispiel 1B. Die im lignocellulosischen Substrat enthaltenen Zucker, 60 g/L Glucose und 32 g/L Xylose, wurden nebeneinander vorliegend in Startvolumen und feed-Lösung im fed-batch-Betrieb unter mikroaeroben Bedingungen mit *Pachysolen tannophilus* (DSMZ, Braunschweig, Deutschland) für 113 Stunden bei 40°C vergoren. Das Startvolumen betrug 300 mL. Das Ergebnis des Experiments ist in Abbildung 5 dargestellt. Die Analysenwerte der Fermentationsbrühen zeigen, dass die Ethanolkonzentration im fed-batch-Betrieb bei 40°C unter 1,5% gehalten werden kann und die simultane Umsetzung sowohl der C6- als auch C5-Zucker mit der Kombination aus fed-batch-Betrieb und in-situ-Gas-Stripping ermöglicht wurde.

### Vergleichsbeispiel 2 - Aufkonzentrierung einer 7,5% (w/v)-Ethanol-Wasser-Lösung durch Gas-Stripping, Adsorption, Desorption und Kondensation

100 mL einer 7,5% (w/v)-Ethanol-Wasser-Lösung wurden für 24 Stunden mit einem Volumenstrom von 0,5 L/min Luft gestrippt. Es wurden eine Membranpumpe (KNF Neuberger, Freiburg, Deutschland), ein Volumenstromregler (Swagelok, Garching, Deutschland) und eine Gaswaschflasche (VWR, Bruchsal, Deutschland) verwendet. Der Gasstrom wurde durch eine Glassäule geleitet (VWR, Bruchsal, Deutschland), welche mit 91 g des Zeolithen (Hergestellt nach US Patent No. 7,244,409 B2) gepackt war. Im Inneren der Säule befanden sich Heizwendeln. Das Gas-Stripping und die Adsorption fanden bei Raumtemperatur statt. Anschließend wurde die Temperatur mit Hilfe der Heizwendeln und über die Säulenwand innerhalb von 90 Minuten linear auf 150°C erhöht. Das desorbierte Ethanol wurde in einer Kühlfalle bei -20°C kondensiert. Der Absolutdruck betrug für Ad- und Desorption jeweils 800 mbar. Der Trägergasstrom wurde im Kreis geführt.

**Ethanol-Konzentrationen:**

| | |
|---|---|
| Ausgangslösung: | 7,48% (w/v) |
| Lösung nach Gas-Stripping: | 2,15% (w/v) |
| Kondensat: | 44,92% (w/v) |

### Vergleichsbeispiel 3 - Selektive Adsorption an Aktivkohle und Zeolith

### A) Gas-Stripping, Adsorption und Desorption mit Zeolith und Vergleich mit Literaturdaten

Das Gas-Stripping und die Adsorption erfolgten genauso wie in Beispiel 2 beschrieben. Allerdings wurde das Volumen der Vorlage auf 1 L erhöht, so dass die Konzentrationsänderung durch die Ethanol-Adsorption relativ gering und damit näherungsweise konstant ist ("steady state"). Die adsorbierte Ethanol-Konzentration wurde durch Gas-Chromatographie (Trace GC, Thermo Fisher) besti m mt.

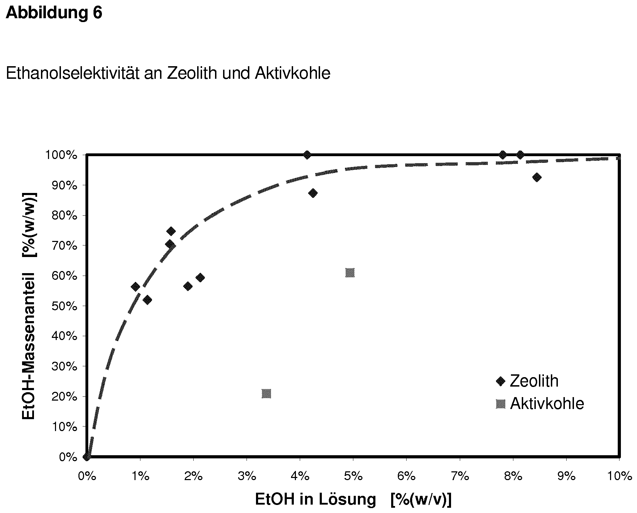

Bei Walsh et al. werden in Table IV, zwei Versuche aufgelistet, bei denen die Ethanol-Konzentration in der Flüssigkeit unter 5% (w/v) lag (4,94% (w/v) und 3,37% (w/v). Berechnet man die Ethanol-Massenanteile aus den adsorbierten Massen, so ergibt sich jeweils 61% (w/w) und 21% (w/w) (siehe Grafik, rote Punkte). Im erfindungsgemäßen Verfahren ergeben sich somit mit dem verwendeten Zeolithen deutlich höhere Ethanol-Massenanteile (siehe Abbildung 6).

### B) Direkter Vergleich von Zeolith mit Aktivkohle bei Gas-Stripping und Adsorption

In zwei ansonsten identischen Versuchen wurde einmal 90 g Zeolith und einmal 90 g Aktivkohle in eine Glassäule (VWR, Bruchsal, Deutschland) gefüllt. Es wurden jeweils 250 mL einer 5% (w/v)-Ethanol-Wasser-Lösung 24 Stunden bei 1 vvm gestrippt. Ansonsten wurde der gleiche Aufbau wie in Beispiel 2 mit einer Membranpumpe (KNF Neuberger, Freiburg, Deutschland), einem Volumenstromregler (Swagelok, Garching, Deutschland) und einer Gaswaschflasche (VWR, Bruchsal, Deutschland) verwendet. Nach 24 Stunden wurde der Versuch beendet, die Gewichtszunahme der Packung bestimmt und die Ethanol-Konzentration durch Gas-Chromatographie (Trace GC, Thermo Fisher) quantifiziert. Da das System geschlossen ist, muss das aus der Lösung gestrippte Ethanol auf dem Zeolithen bzw. der Aktivkohle adsorbiert worden sein. Die restliche Gewichtszunahme ist auf Wasser zurückzuführen. Über eine Massenbilanz wurden daher die adsorbierten Mengen von Ethanol und Wasser berechnet und somit folgende Ethanol-Massenanteile der adsorbierten Mischung bestimmt:

| | |
|---|---|
| Zeolith: | 97,4% (w/w) |
| Aktivkohle: | 49,8% (w/w) |

Es zeigt sich also, dass die Verwendung eines Zeolithen deutliche Vorteile gegenüber Aktivkohle aufweist, da die Adsorption bei Zeolithen deutlich selektiver abläuft. Dies ist in Hinblick auf die Energiekosten für die nachfolgende thermische Aufreinigung von entscheidendem Vorteil.

Mit Aktivkohle kann der technische Effekt der vorliegenden Anmeldung nicht erzielt werden, denn Aktivkohle bindet deutlich mehr Wasser als Zeolith. Der entscheidende Vorteil von Zeolith gegenüber Aktivkohle ist also die höhere Selektivität, d.h. Zeolith bindet bevorzugt Ethanol und nur wenig Wasser. Dadurch ist die nachfolgende Aufreinigung des desorbierten Ethanols vereinfacht (weniger Masse muss desorbiert werden, die Rektifikationssäule ist kleiner und die Energiekosten sind drastisch reduziert).

## Patentansprüche

1. Verfahren zur Ethanol-Herstellung umfassend:
a. die fermentative Umsetzung von C5 und/oder C6-Zuckern zu Ethanol in einer Fermentationslösung;
b. die *in situ*-Entfernung des Ethanols durch Gas-Stripping mit Hilfe eines Trägergases, wobei die Ethanol-Konzentration in der Fermentationslösung unter 5% (w/v) gehalten wird und wobei das Gas-Stripping in einer mit dem Fermenter verbundenen Gas-Stripping-Säule durchgeführt wird, die kontinuierlich mit der Fermentationslösung gespeist wird und deren Auslass wieder zurück in den Fermenter geführt wird;
c. Durchleiten des durch Gas-Stripping entstandenen Ethanol-Trägergas-Gemisches durch einen Zeolith-Adsorber, wobei Ethanol aus dem Gasgemisch an einen Adsorber adsorbiert wird, und wobei mehrere Ad-/Desorptionssäulen parallel und / oder in Reihe geschaltet werden und die Adsorption in einer Säule zugleich mit der Desorption in einer anderen Säule durchgeführt wird; und
d. die Desorption des adsorbierten Ethanols von dem Adsorber;
wobei das Trägergas nach dem Austritt aus dem Adsorber in die in der Gas-Stripping-Säule enthaltene Fermentationslösung rückgeführt wird.

2. Verfahren nach Anspruch 1, wobei in der Fermentationslösung vorhandene C5-Zucker zu Ethanol umgesetzt werden.

3. Verfahren nach einem oder mehreren der Ansprüche 1 und 2, wobei die Menge adsorbierten Ethanols am Ende der Fermentation mindestens 20% der maximalen Ethanolaufnahmemenge des Adsorbers beträgt.

4. Verfahren nach einem oder mehreren der Ansprüche 1 bis 3, wobei die Adsorption bei einer Temperatur erfolgt, die nicht über der Temperatur des Ethanol-Trägergas-Gemisches bei Austritt aus der Fermentationslösung liegt.

5. Verfahren nach einem oder mehreren der vorhergehenden Ansprüche, wobei der Zeolith-Adsorber ein SiO₂/Al₂O₃-Verhältnis über 200 und unter 1000 aufweist.

6. Verfahren nach Anspruch 5, wobei die Gas-Stripping-Säule im Gegenstrom betrieben wird und / oder Füllkörper enthält.

7. Verfahren nach einem oder mehreren der vorhergehenden Ansprüche, wobei die spezifische Begasungsrate zwischen 0,1 und 10 vvm liegt.

8. Verfahren nach einem oder mehreren der vorhergehenden Ansprüche, wobei Kohlenstoffdioxid, welches während der Fermentation gebildet wird, als Trägergasstrom eingesetzt wird.

9. Verfahren gemäß einem oder mehreren der vorhergehenden Ansprüche, wobei in der Ad-/ Desorptionssäule der Ein- und Austrag von thermischer Energie zusätzlich zur Säulenwand und durch den Trägergasstrom auch über Heizwendel erfolgt.

10. Verfahren gemäß einem oder mehreren der vorhergehenden Ansprüche, wobei die Temperatur bei der Adsorption des Ethanols zwischen 10-100°C und der Druck zwischen 0,5 und 10 bar liegt.

11. Verfahren gemäß einem oder mehreren der vorangehenden Ansprüche, wobei die Ad-/Desorptionssäulen mit unterschiedlichen Arten von Adsorbentien gefüllt sind.

## Claims

1. Process for ethanol production comprising:
a. fermentative conversion of C5 and/or C6 sugars to ethanol in a fermentation solution;
b. in situ removal of the ethanol by gas stripping using a carrier gas, wherein the ethanol concentration in the fermentation solution is kept below 5% (w/v) and wherein the gas stripping is performed in a gas stripping column connected to the fermenter which is continuously fed with the fermentation solution and whose discharge is sent back into the fermenter;
c. passing the ethanol-carrier gas mixture formed by gas stripping through a zeolite adsorber, wherein ethanol from the gas mixture is adsorbed onto an adsorber and wherein a plurality of adsorption/desorption columns are connected in parallel and/or in series and adsorption in one column is performed simultaneously with desorption in another column; and
d. desorption of the adsorbed ethanol from the adsorber;
wherein after issuing from the adsorber the carrier gas is recycled into the fermentation solution contained in the gas stripping column.

2. Process according to Claim 1, wherein C5 sugars present in the fermentation solution are converted to ethanol.

3. Process according to one or more of Claims 1 and 2, wherein the amount of adsorbed ethanol at the end of the fermentation is at least 20% of the maximum ethanol uptake amount for the adsorber.

4. Process according to one or more of Claims 1 to 3, wherein the adsorption is effected at a temperature not higher than the temperature of the ethanol-carrier gas mixture upon issuance from the fermentation solution.

5. Process according to one or more of the preceding claims, wherein the zeolite adsorber has a SiO₂/Al₂O₃ ratio above 200 and below 1000.

6. Process according to Claim 5, wherein the gas stripping column is operated in countercurrent and/or contains packing elements.

7. Process according to one or more of the preceding claims, wherein the specific gas supply rate is between 0.1 and 10 vvm.

8. Process according to one or more of the preceding claims, wherein carbon dioxide formed during the fermentation is employed as the carrier gas stream.

9. Process according to one or more of the preceding claims, wherein in addition to the column wall and through the carrier gas stream, the supply and removal of thermal energy is also effected via heating coils in the adsorption/desorption column.

10. Process according to one or more of the preceding claims, wherein the temperature during the adsorption of the ethanol is between 10-100°C and the pressure is between 0.5 and 10 bar.

11. Process according to one or more of the preceding claims, wherein the adsorption/desorption columns are filled with different types of adsorbents.

## Revendications

1. Procédé de fabrication d'éthanol, comprenant :
a. la transformation fermentative de sucres en C5 et/ou C6 en éthanol dans une solution de fermentation ;
b. l'élimination *in situ* de l'éthanol par extraction gazeuse à l'aide d'un gaz vecteur, la concentration d'éthanol dans la solution de fermentation étant maintenue en dessous de 5 % (m/v) et l'extraction gazeuse étant réalisée dans une colonne d'extraction gazeuse raccordée au fermentateur, qui est alimentée en continu avec la solution de fermentation et dont la sortie est réintroduite dans le fermentateur ;
c. le passage du mélange éthanol-gaz vecteur formé par l'extraction gazeuse dans un adsorbeur à zéolithe, l'éthanol du mélange gazeux étant adsorbé sur un adsorbeur et plusieurs colonnes d'ad-/désorption étant raccordées en parallèle et/ou en série et l'adsorption dans une colonne étant réalisée simultanément à la désorption dans une autre colonne ; et
d. la désorption de l'éthanol adsorbé de l'adsorbeur ; le gaz vecteur étant recyclé dans la solution de fermentation contenue dans la colonne d'extraction gazeuse après la sortie de l'adsorbeur.

2. Procédé selon la revendication 1, dans lequel des sucres en C5 présents dans la solution de fermentation sont transformés en éthanol.

3. Procédé selon une ou plusieurs des revendications 1 et 2, dans lequel la quantité d'éthanol adsorbé à la fin de la fermentation est d'au moins 20 % de la quantité maximale de fixation d'éthanol de l'adsorbeur.

4. Procédé selon une ou plusieurs des revendications 1 à 3, dans lequel l' adsorption a lieu à une température qui n'est pas supérieure à la température du mélange éthanol-gaz vecteur lors de la sortie de la solution de fermentation.

5. Procédé selon une ou plusieurs des revendications précédentes, dans lequel l'adsorbeur à zéolithe présente un rapport SiO₂/Al₂O₃ supérieur à 200 et inférieur à 1 000.

6. Procédé selon la revendication 5, dans lequel la colonne d'extraction gazeuse est exploitée à contre-courant et/ou contient des corps de remplissage.

7. Procédé selon une ou plusieurs des revendications précédentes, dans lequel le taux de gazage spécifique est compris entre 0,1 et 10 vvm.

8. Procédé selon une ou plusieurs des revendications précédentes, dans lequel du dioxyde de carbone qui est formé pendant la fermentation est utilisé en tant que courant de gaz vecteur.

9. Procédé selon une ou plusieurs des revendications précédentes, dans lequel l'entrée et la sortie d'énergie thermique dans la colonne d'ad-/désorption ont lieu en plus de la paroi de la colonne et du courant de gaz vecteur également par une résistance hélicoïdale chauffante.

10. Procédé selon une ou plusieurs des revendications précédentes, dans lequel la température lors de l'adsorption de l'éthanol est comprise entre 10 et 100 °C et la pression entre 0,5 et 10 bar.

11. Procédé selon une ou plusieurs des revendications précédentes, dans lequel les colonnes d'ad-/désorption sont remplies avec différents types d'adsorbants.
